# EUROPÄISCHE PATENTSCHRIFT

(11) **EP 1 263 922 B1**
(45) Veröffentlichungstag und Bekanntmachung des Hinweises auf die Patenterteilung: **16.06.2004**
(21) Anmeldenummer: 01927697.1
(22) Anmeldetag: 23.02.2001
(51) Int. Cl.: C11D 17/00, C11D 3/02, A61K 9/20, A61K 7/00, A01N 25/34, C05G 3/00, C02F 1/00

(54) **VERFAHREN ZUR HERSTELLUNG VON FORMKÖRPERN**
METHOD FOR PRODUCING MOULDED BODIES
PROCEDE POUR PRODUIRE DES CORPS MOULES

(30) Priorität: 04.03.2000 DE 10010759
(43) Veröffentlichungstag der Anmeldung: 11.12.2002
(73) Patentinhaber: Henkel Kommanditgesellschaft auf Aktien, 40589 Düsseldorf (DE)
(72) Erfinder: SCHMIEDEL, Peter, 40599 Düsseldorf (DE)
(86) Internationale Anmeldenummer: PCT/EP2001/002074
(87) Internationale Veröffentlichungsnummer: WO 2001/066684

(56) Entgegenhaltungen:
- WO-A-95/07976
- US-A- 3 489 687
- DATABASE WPI Section Ch, Week 198638 Derwent Publications Ltd., London, GB; Class A96, AN 1986-248712 XP002174367 & JP 61 176519 A (KAO CORP), 8. August 1986 (1986-08-08)
- DATABASE WPI Section Ch, Week 198251 Derwent Publications Ltd., London, GB; Class D15, AN 1982-10378J XP002174368 & JP 57 184491 A (RYONICHI KK), 13. November 1982 (1982-11-13)
- DATABASE WPI Section Ch, Week 198110 Derwent Publications Ltd., London, GB; Class B07, AN 1981-16266D XP002174369 & JP 55 167039 A (TAIYO CHEM IND CO LTD), 26. Dezember 1980 (1980-12-26)

## Beschreibung

Die vorliegende Erfindung betrifft ein Verfahren zur Herstellung von Formkörpern, z.B. zur Herstellung von Wasch- und Reinigungsmittelformkörpern, Formkörpern zur Herstellung wassriger Bindemittelsysteme (Tapetenkleistertablette), Formkörpern für pharmazeutische oder kosmetische Anwendungen oder Formkörper für den Agrarbereich. enthaltend einen oder mehrere Aktivstoffe, worin die Ausgangskomponenten in Form gebracht und anschließend verfestigt werden.

Tabletten, auch als Formkörper bezeichnet, sind in der Pharmazie und in der chemischen Technologie eine vielfach eingesetzte Erzeugnisform. Üblicherweise bestehen sie aus einer aus mehrerer Aktivsubstanz(en) (z.B. Arzneistoff) und gegebenenfalls Zusätze von Füll-. Binde- und Gleitmitteln. Ein weiteres Einsatzgebiet sind auch die Wasch- und Reinigungsmittel.

Die tablettierten Erzeugnisse haben gegenüber den ursprünglich ungeformten Bestandteiien eine Reihe von Vorteilen: sie sind einfacher zu dosieren und zu handhaben und haben aufgrund ihrer kompakten Struktur Vorteile bei der Lagerung und beim Transport. Die Herstellung der Tabletten erfolgt in der Regel in speziellen Tablettiermaschinen mit Hilfe von Exzenter- und Rundlaufpressen. Die ungeformten Ausgangsbestandteile, häufig Pulver. werden entweder direkt verpresst oder aber vorher granuliert, um das Fließ- und Haftverhalten der Ausgangskomponenten zu verbessern.

Das Herstellen von Formkörpern über Tablettiermaschinen ist sehr aufwendig, da insbesondere druckempfindliche Komponenten nur in begrenztem Maße eingesetzt werden können. Eine weitere Möglichkeit, Formkörper herzustellen, ist es die Inhaltsstoffe in Form von Schmelzen zu verarbeiten, z.B. ein schmelzbares Material als Matrixmaterial für die weiteren Inhaltsstoffe zu verwenden und anschließend die Schmelze formgebend zu verarbeiten. Auch das Verarbeiten in einer Schmelze hat den Nachteil, dass temperaturempfindliche Inhaltsstoffe nicht oder nur in geringem Maße eingearbeitet werden können. Außerdem zeigen derartig hergestellte Formkörper oftmals ein unbefriedigendes Zerfallsverhalten.

Der vorliegenden Erfindung lag die Aufgabe zugrunde, ein einfaches Verfahren zur Herstellung von Formkörpern, insbesondere von Wasch- und Reinigungsmittelformkörpern. Formkörper für die Herstellung von Bindemittelsystemen oder Formkörpern für kosmetische Anwendungen sowie für den Agrarbereich zur Verfügung zu stellen, worin die Formkörper nahezu ohne thermische oder Druckbelastung erhalten werden können.

Gegenstand der vorliegenden Erfindung ist ein Verfahren zur Herstellung von Formkörpern enthaltend einen oder mehrere Aktivstoffe, gemäß Anspruch 1.

Bei der Durchführung des erfindungsgemäßen Verfahrens reagieren die Komponenten A und B unter Verfestigung der einzelnen Inhaltsstoffe miteinander. Das gebildete Reaktionsprodukt aus den Komponenten A und B verbindet die einzelnen Ausgangskomponenten derart, dass ein fester, relativ bruchstabiler Formkörper erhalten wird.

Mit dem erfindungsgemäßen Verfahren werden Formkörper mit einem guten Zerfall erhalten. Da die Bindung der einzelnen Inhaltsstoffe durch eine reaktive Sinterung erfolgt und nicht durch die "Klebrigkeit" der Granula des Vorgemisches bedingt ist, ist es nicht notwendig, die Rezeptur an die Bindeeigenschaften der einzelnen Inhaltsstoffe anzupassen. Diese können beliebig in Abhängigkeit von ihrer Wirksamkeit angepasst werden.

Um die Komponenten A und B miteinander zur Reaktion zu bringen, hat es sich als vorteilhaft erwiesen, wenn die Ausgangskomponenten mit der Komponente A vermischt oder bevor sie in Form gebracht werden damit beschichtet werden. Beispiele für Verbindungen der Komponente A sind die Alkalihydroxide, insbesondere NaOH und KOH, Erdalkalihydroxide, insbesondere Ca(OH)₂. Alkalisilikate organische oder anorganische Säuren, wie Zitronensäure, oder saure Salze wie Hydrogensulfat, wasserfreie hydratisierbare Salze oder Hydratwasser-haltige Salze, wie Soda, Acetate, Sulfate, Alkalimetallate, wobei die voran genannten Verbindungen. sofern möglich. auch in Form ihrer wässerigen Lösungen eingesetzt werden können.

Die Komponente B ist derart ausgewählt, dass sie mit der Komponente A ohne Ausübung von höheren Drücken oder wesentlicher Temperaturerhöhung unter Ausbildung eines Feststoffes unter Verfestigung der weiteren vorhandenen Ausgangskomponenten reagiert. Beispiele für Verbindungen der Komponente B sind CO₂, NH₃, Wasserdampf oder Sprühnebel, Hydratwasser-haltige Salze, welche gegebenenfalls mit den als Komponente A vorliegenden wasserfreien Salzen durch Hydratwanderung reagieren, Hydrate bildende wasserfreie Salze, die mit den Hydratwasser-haltigen Salzen der Komponente A unter Hydratwanderung reagieren, SO₂, SO₃, HCl, HBr.

Die oben genannten Komponenten A und B sind untereinander austauschbar, sofern zwei Komponenten eingesetzt werden, die unter Sinterung miteinander reagieren.

In einer bevorzugten Ausführungsform der vorliegenden Erfindung werden die Ausgangskomponenten mit Verbindungen der Komponente A vermischt oder beschichtet und anschließend mit den Verbindungen der Komponente B versetzt. Es hat sich als besonderes geeignet erwiesen, wenn die Verbindungen der Komponente B gasförmig sind. Die in Form gebrachten Ausgangskomponenten (im Folgenden als Vorformlinge bezeichnet) können dann entweder in einfacher Form begast oder in eine Gasatmosphäre eingebracht werden. Eine besonders bevorzugte Kombination aus den Komponenten A und B sind Alkalihydroxide, insbesondere NaOH und KOH, Erdalkalhyddroxide, oder Alkalisilikate, die vorzugweise in Form von wässrigen Lösugnen eingesetzt werden, als Komponente A und CO₂ als Komponente B.

Zur Durchführung des erfindungsgemäßen Verfahrens werden die Ausgangskomponenten zunächst in Form gebracht, d.h. sie werden üblicherweise in eine Matrize, die die äußere Form des herzustellenden Formkörpers aufweist, gefüllt. Die Ausgangskomponenten liegen vorzugsweise in pulvriger bis granularer Form vor. Zunächst werden sie mit der Komponente A vermischt oder beschichtet. Nach dem Einfüllen in die Matrize beziehungsweise Tablettenform hat es sich erwiesen, die Ausgangskomponenten in der Matrize leicht anzudrücken. z.B. mit der Hand oder mit einem Stempel bei einem Druck bis zu 1 kN, oder das Vorgemisch durch Vibration zu verdichten (Klopfverdichtung).

Anschließend werden sie, sofern die Komponente A nicht bereits im Gemisch mit den Ausgangskomponenten vorliegt, damit beschichtet und mit der Komponente B versetzt.

Nach Ablauf der Reaktion wird ein bruchstabiler Formkörper ohne Einwirkung von Druck oder Temperatur erhalten.

Ist eine der Komponenten A oder B ein Gas, so kann ein Vorformling z.B. mit diesem versetzt werden, so dass das Gas diesen durchströmt. Diese Verfahrensführung ermöglicht ein gleichmäßiges Erhärten des Formkörpers innerhalb kurzer Zeit.

In einer weiteren Verfahrensvariante wird ein Vorformling in eine Atmosphäre des reaktiven Gases eingebracht. Diese Variante ist einfach durchzuführen. Es ist möglich, Formkörper herszustellen, die einen Härtegradienten aufweisen, d.h. Formkörper, die nur eine gehärtere Oberfläche aufweisen bis hin zu Formkörper die vollständig ausgehärtet sind.

Ein Vorformling bzw. das Vorgemisch kann auch unter Überdruck mit dem reaktiven Gas umgesetzt werden. Diese Verfahrensvariante hat den Vorteil, dass die Oberfläche schnell unter Bildung einer harten Schale aushärtet, wobei der Härtungsprozess hier bereits gestoppt werden oder wie voranstehend beschrieben über steigende Härtungsstufen können auch vollständig ausgehärtete Formkörper hergestellt werden.

Die voranstehenden Verfahrensvarianteh könne auch kombiniert werden. indem man zunächst reaktives Gas durch den Vorformling strömen lässt, um Luft zu verdrängen. Anschließend setzt man den Vorformling einer Gasatmosphäre bei Normaldruck aus. Durch die Reaktion zwischen dem Gas und der zweiten Komponente wird automatisch Gas in den Formling hineingesaugt.

In einer möglichen Ausführungsform der vorliegenden Erfindung wird nicht das Ausgangsgemisch sondern ein bereits in Form gebrachtes Vorformling mit der Komponente A beschichtet und anschließend mit der Komponente B zur Reaktion gebracht. Es härtet die sich an der Oberfläche des Vorformlings befindenden Schicht aus, während im Kern die lockere bzw. leicht verdichtete Struktur erhalten bleibt. Derartige Formkörper zeichnen sich durch ein besonders gutes Zerfallsverhalten aus.

Die Festigkeit der erfindungsgemäß hergestellten Formkörper lässt sich durch eine geeignete Auswahl der Komponenten A und B und auch durch die eingesetzte Menge dieser Komponenten einstellen. Darüberhinaus können die Formkörper Bindemittel enthalten, obwohl ihr Einsatz nicht bevorzugt ist. Als Bindemittel kommen eine Vielzahl von aus der Pharmazie bekannten Bindemitteln für Tabletten in Frage. Sie unterscheiden sich durch ihre Hydrophilie/Hydrophobie, ihre Löslichkeit und daraus resultierend durch ihre Bindewirkung. Insbesondere können Stoffe aus der Gruppe der Polyalkylenglycole und Polyoxyalkylenglycole unterschiedlichen Molekulargewichtes eingesetzt werden. Besonders bewährt haben sich. Daneben sind auch andere Stoffe geeignet. wie z.B. Wachse, Paraffine, Fettsäuresalze (Seifen), insbesondere Stearate: Fettsäuren und Fettalkohole, Fettsäureester, Cellulosederivate, Hydrokolloide, Mono-. Oligo-, oder Polysaccharide und Polymere Verbindungen (z.B. Polyacrylate) und Harze.

Um den späteren Zerfall der Formkörper bei der Anwendung zu beschleunigen können sie optional sogenannte Tablettensprengmittel, auch Desintegrationsmittel genannt enthalten. Ein großer Vorteil der nach dem erfindungsgemäßen Verfahren hergestellten Formkörper besteht jedoch darin. dass im Vergleich zu verpressten Formkörpern gleicher Härte auch ohne den Einsatz eines Sprengmittels oder mit einer geringeren Menge gute Zerfallsergebnisse erreicht werden.

Die erfindungsgemäß hergestellten Formkörper können für eine Vielzahl von Anwendungen eingesetzt werden. In Abhängigkeit von der gewählten Anwendung werden die Ausgangskomponenten ausgewählt. Als mögliche Anwendungen können Waschmittel, Reinigungsmittel, insbesondere Geschirrspülmittel für maschinelles, aber auch manuelles Spülen, Tabletten für kosmetische Anwendungen, Tapetenkleistertabletten und Tabletten für den Einsatz im Agrarbereich (Pestizide, Herbizide...) genannt werden.

Das erfindungsgemäße Verfahren ermöglicht die Herstellung von ein- und mehrphasigen Formkörpern, die beliebige Formen aufweisen können. Zur Herstellung von mehrphasigen Formkörpern hat es sich als vorteilhaft erwiesen, zunächst einen Vorformling aus einer Phase herzustellen. Anschließend können eine oder mehrere weitere Phasen aufgebracht werden. Die Phasen können sich durch verschiedenartige Inhaltsstoffe und auch nur durch ihre Färbung unterscheiden. Durch diese Verfahrensweise. insbesondere durch die Einsatzmenge der Komponente A in den jeweiligen Phasen können Phasen mit unterschiedlicher Härte und Zerfallszeit hergestellt werden. so dass ihre Wirkstoffe währen der Anwendung kontrolliert sequentiell freigesetzt werden können.

In einer weiteren Ausführungsform weisen die erfindungsgemäßen Formkörper eine Kavität aufweist, die entsprechend befüllt werden kann. Als Füllungen für die Kavität kommen z.B. eine erstarrende Schmelze oder auch Pulver in Betracht, wobei das Pulver mittels einer Coatingschicht in der Kavität befestigt werden kann. Als Inhaltsstoffe werden in die Kaviät vorzugsweise Substanzen eingearbeitet, die bei der Anwendung der Formkörper ggf. zu einem späteren oder früheren Zeitpunkt als die Inhaltsstoffe des gesinterten Formkörperteils freigesetzt werden können.

Neben dem Eindosieren einer Schmelze können auch ein oder mehrere anderweitig, bevorzugt durch ein nichtkomprimierendes Verfahren vorab hergestellte(r) Kem(e) beliebiger Gestalt, z.B. Kugel, Ellipsoid, Linse montiert werden. Die Montage kann dabei erfolgen, indem der Kern in den erhärteten Formkörper eingeklebt wird oder in den Vorformling eingebracht wird und sich während der Erhärtungsphase form- oder reibschlüssig mit diesem verbindet. Der Kern muss dabei nicht unbedingt an der Oberfläche des Formkörpers sichtbar sein. Er kann sich auch vollständig unterhalb der Oberfläche oder genau im Zentrum des gesinterten Formkörpers befinden.

Weiterhin kann der Kern lose in die Kavität an der Oberfläche des bevorzugt bereits ausgehärteten Formkörpers eingelegt und durch übergießen mit einem Klebstoff oder einer Schmelze, z.B. einer wachsartigen Substanz, wie Paraffin, PEG etc., fixiert werden.

In einer bevorzugten Ausführungsform ist der erfindungsgemäß hergestellte Formkörper ein maschinelles Geschirrspülmittel oder Textilwaschmittel. In dieser Ausgestaltung sind die Aktivstoffe vorzugsweise ausgewählt aus Buildermaterialien, Tensiden, Bleichmitteln, Bleichaktivatoren, Enzymen, Enzymstabilisatoren, Korrosionsinhibitoren, Belagsinhibitoren, Komplexbildnern, anorganischen Salzen, Vergrauungsinhibitoren, Schauminhibitoren, Silikonölen, Soil-release-Verbindungen, Farbübertragungsinhibitoren, Salzen von Polyphosphonsäuren, optischen Aufhellern, Fluoreszenzmitteln, Desinfektionsmitteln, Duftstoffen, Farbstoffen, Antistatika, Bügelhilfsmitteln, Phobier- und Imprägniermitteln, Quell- und Schiebefestmitteln, UV-Absorbern oder deren Gemische.

Als Buildermaterialien können alle auf dem Gebiet der Wasch- und Reinigungsmittel bekannten Buildersubstanzen eingesetzt werden. insbesondere die Zeolithe, Silikate, Carbonate, Phosphate sowie organische Cobuilder.

Geeignete kristalline, schichtförmige Natriumsilikate besitzen die allgemeine Formel NaMSiₓO₂ₓ₊₁. H₂O, wobei M Natrium oder Wasserstoff bedeutet, x eine Zahl von 1,9 bis 4 und y eine Zahl von 0 bis 20 ist und bevorzugte Werte für x 2, 3 oder 4 sind. Bevorzugte kristalline Schichtsilikate der angegebenen Formel sind solche. in denen M für Natrium steht und x die Werte 2 oder 3 annimmt. Insbesondere sind sowohl β- als auch δ-Natriumdisilikate Na₂Si₂O₅. yH2O bevorzugt.

Einsetzbar sind auch amorphe Natriumsilikate mit einem Modul Na₂O : SiO₂ von 1:2 bis 1:3.3. vorzugsweise von 1:2 bis 1:2,8 und insbesondere von 1:2 bis 1:2,6, welche löseverzögert sind und Sekundärwascheigenschaften aufweisen. Die Löseverzögerung gegenüber herkömmlichen amorphen Natriumsilikaten kann dabei auf verschiedene Weise, beispielsweise durch Oberflächenbehandlung. Compoundierung, Kompaktierung/ Verdichtung oder durch Übertrocknung hervorgerufen worden sein. Im Rahmen dieser Erfindung wird unter dem Begriff "amorph" auch "röntgenamorph" verstanden. Dies heißt. dass die Silikate bei Röntgenbeugungsexperimenten keine scharfen Röntgenreflexe liefern. wie sie für kristalline Substanzen typisch sind, sondern allenfalls ein oder mehrere Maxima der gestreuten Röntgenstrahlung, die eine Breite von mehreren Gradeinheiten des Beugungswinkels aufweisen. Es kann jedoch sehr wohl sogar zu besonders guten Buildereigenschaften führen, wenn die Silikatpartikel bei Elektronenbeugungsexperimenten verwaschene oder sogar scharfe Beugungsmaxima liefern. Dies ist so zu interpretieren, dass die Produkte mikrokristalline Bereiche der Größe 10 bis einige Hundert nm aufweisen, wobei Werte bis max. 50 nm und insbesondere bis max. 20 nm bevorzugt sind. Insbesondere bevorzugt sind verdichtete/kompaktierte amorphe Silikate, compoundierte amorphe Silikate und übertrocknete röntgenamorphe Silikate.

Der eingesetzte feinkristalline, synthetische und gebundenes Wasser enthaltende Zeolith ist vorzugsweise Zeolith A und/oder P. Als Zeolith P wird Zeolith MAP® (Handelsprodukt der Firma Crosfield) besonders bevorzugt. Geeignet sind jedoch auch Zeolith X sowie Mischungen aus A, X und/oder P. Kommerziell erhältlich und im Rahmen der vorliegenden Erfindung bevorzugt einsetzbar ist beispielsweise auch ein Co-Kristallisat aus Zeolith X und Zeolith A (ca. 80 Gew.-% Zeolith X). das von der Firma CONDEA Augusta S.p.A. unter dem Markennamen VEGOBOND AX® vertrieben wird und durch die Formel

nNa₂O . (1-n)K₂O . Al₂O₃ . (2 - 2,5)SiO₂ . (3.5 - 5,5) H₂O

beschrieben werden kann. Geeignete Zeolithe weisen eine mittlere Teilchengröße von weniger als 10 um (Volumenverteilung; Messmethode: Coulter Counter) auf und enthalten vorzugsweise 18 bis 22 Gew.-%, insbesondere 20 bis 22 Gew.-% an gebundenem Wasser.

Selbstverständlich ist auch ein Einsatz der allgemein bekannten Phosphate als Buildersubstanzen möglich. Unter der Vielzahl der kommerziell erhältlichen Phosphate haben die Alkalimetallphosphate unter besonderer Bevorzugung von Pentanatrium- bzw. Pentakaliumtriphosphat (Natrium- bzw. Kaliumtripolyphosphat) in der Wasch- und Reinigungsmittel-Industrie die größte Bedeutung.

Als organische Cobuilder können in den erfindungsgemäßen maschinellen Geschirrspülmitteln insbesondere Polycarboxylate/Polycarbonsäuren, polymere Polycarboxylate, Asparaginsäure, Polyacetale, Dextrine. weitere organische Cobuilder (siehe unten) sowie Phosphonate eingesetzt werden. Diese Stoffklassen werden nachfolgend beschrieben.

Geeignete organische Gerüstsubstanzen sind beispielsweise die in Form ihrer Natriumsalze einsetzbaren Polycarbonsäuren, wobei unter Polycarbonsäuren solche Carbonsäuren verstanden werden, die mehr als eine Säurefunktion tragen. Beispielsweise sind dies Citronensäure, Adipinsäure, Bernsteinsäure, Glutarsäure, Äpfelsäure, Weinsäure, Maleinsäure, Fumarsäure, Zuckersäuren, Aminocarbonsäuren, Nitrilotriessigsäure (NTA), sofern ein derartiger Einsatz aus ökologischen Gründen nicht zu beanstanden ist, sowie Mischungen aus diesen. Bevorzugte Salze sind die Salze der Polycarbonsäuren wie Citronensäure, Adipinsäure, Bernsteinsäure, Glutarsäure, Weinsäure,' Zuckersäuren und Mischungen aus diesen.

Auch die Säuren an sich können eingesetzt werden. Die Säuren besitzen neben ihrer Builderwirkung typischerweise auch die Eigenschaft einer Säuerungskomponente und dienen somit auch zur Einstellung eines niedrigeren pH-Wertes von Wasch- oder Reinigungsmitteln. Insbesondere sind hierbei Citronensäure, Bernsteinsäure. Glutarsäure, Adipinsäure, Gluconsäure und beliebige Mischungen aus diesen zu nennen.

Als Builder sind weiter polymere Polycarboxylate geeignet, dies sind beispielsweise die Alkalimetallsalze der Polyacrylsäure oder der Polymethacrylsäure. beispielsweise solche mit einer relativen Molekülmasse von 500 bis 70000 g/mol.

Bei den für polymere Polycarboxylate angegebenen Molmassen handelt es sich im Sinne dieser Schrift um gewichtsmittlere Molmassen M_{w} der jeweiligen Säureform, die grundsätzlich mittels Gelpermeationschromatographie (GPC) bestimmt wurden, wobei ein UV-Detektor eingesetzt wurde. Die Messung erfolgte dabei gegen einen externen Polyacrylsäure-Standard, der aufgrund seiner strukturellen Verwandtschaft mit den untersuchten Polymeren realistische Molgewichtswerte liefert. Diese Angaben weichen deutlich von den Molgewichtsangaben ab, bei denen Polystyrolsulfonsäuren als Standard eingesetzt werden. Die gegen Polystyrolsulfonsäuren gemessenen Molmassen sind in der Regel deutlich höher als die in dieser Schrift angegebenen Molmassen.

Geeignete Polymere sind insbesondere Polyacrylate, die bevorzugt eine Molekülmasse von 2000 bis 20000 g/mol aufweisen. Aufgrund ihrer überlegenen Löslichkeit können aus dieser Gruppe wiederum die kurzkettigen Polyacrylate, die Molmassen von 2000 bis 10000 g/mol, und besonders bevorzugt von 3000 bis 5000 g/mol, aufweisen, bevorzugt sein.

Geeignet sind weiterhin copolymere Polycarboxylate, insbesondere solche der Acrylsäure mit Methacrylsäure und der Acrylsäure oder Methacrylsäure mit Maleinsäure. Als besonders geeignet haben sich Copolymere der Acrylsäure mit Maleinsäure erwiesen, die 50 bis 90 Gew.-% Acrylsäure und 50 bis 10 Gew.-% Maleinsäure enthalten. Ihre relative Molekülmasse, bezogen auf freie Säuren, beträgt im allgemeinen 2000 bis 70000 g/mol, vorzugsweise 20000 bis 50000 g/mol und insbesondere 30000 bis 40000 g/mol.

Die (co-)polymeren Polycarboxylate können entweder als Pulver oder als wässrige Lösung eingesetzt werden. Der Gehalt der Mittel an (co-)polymeren Polycarboxylaten beträgt vorzugsweise 0,5 bis 20 Gew.-%, insbesondere 3 bis 10 Gew.-%.

Zur Verbesserung der Wasserlöslichkeit können die Polymere auch Allylsulfonsäuren, wie beispielsweise Allyloxybenzolsulfonsäure und Methallylsulfonsäure, als Monomer enthalten.

Insbesondere bevorzugt sind auch biologisch abbaubare Polymere aus mehr als zwei verschiedenen Monomereinheiten, beispielsweise solche, die als Monomere Salze der Acrylsäure und der Maleinsäure sowie Vinylalkohol bzw. Vinylalkohol-Derivate oder die als Monomere Salze der Acrylsäure und der 2-Alkylallylsulfonsäure sowie Zucker-Derivate enthalten.

Weitere bevorzugte Copolymere sind solche. die als Monomere vorzugsweise Acrolein und Acrylsäure/Acrylsäuresalze bzw. Acrolein und Vinylacetat aufweisen.

Ebenso sind als weitere bevorzugte Buildersubstanzen polymere Aminodicarbonsäuren, deren Salze oder deren Vorläufersubstanzen zu nennen. beispielsweise Polyasparaginsäuren bzw. deren Salze und Derivate.

Weitere geeignete Buildersubstanzen sind Polyacetale, welche durch Umsetzung von Dialdehyden mit Polyolcarbonsäuren, welche 5 bis 7 C-Atome und mindestens 3 Hydroxylgruppen aufweisen. erhalten werden können. Bevorzugte Polyacetale werden aus Dialdehyden wie Glyoxal, Glutaraldehyd, Terephthalaldehyd sowie deren Gemischen und aus Polyolcarbonsäuren wie Gluconsäure und/oder Glucoheptonsäure erhalten.

Weitere geeignete organische Buildersubstanzen sind Dextrine, beispielsweise Oligomere bzw. Polymere von Kohlenhydraten, die durch partielle Hydrolyse von Stärken erhalten werden können. Die Hydrolyse kann nach üblichen. beispielsweise säure- oder enzymkatalysierten Verfahren durchgeführt werden Vorzugsweise handel es sich um Hydrolyseprodukte mit mittleren Molmassen im Bereich von 400 bis 500000 g/mol. Dabei ist ein Polysaccharid mit einem Dextrose-Äquivalent (DE) im Bereich von 0,5 bis 40, insbesondere von 2 bis 30 bevorzugt. wobei DE ein gebräuchliches Maß für die reduzierende Wirkung eines Polysaccharids im Vergleich zu Dextrose, welche ein DE von 100 besitzt, ist. Brauchbar sind sowohl Maltodextrine mit einem DE zwischen 3 und 20 und Trockenglucosesirupe mit einem DE zwischen 20 und 37 als auch sogenannte Gelbdextrine und Weißdextrine mit höheren Molmassen im Bereich von 2000 bis 30000 g/mol.

Bei den oxidierten Derivaten derartiger Dextrine handelt es sich um deren Umsetzungsprodukte mit Oxidatiorasmittein, welche in der Lage sind, mindestens eine Alkoholfunktion des Saccharidrings zur Carbonsäurefunktion zu oxidieren. Ein an C₆ des Saccharidrings oxidiertes Produkt kann besonders vorteilhaft sein.

Auch Oxydisuccinate und andere Derivate von Disuccinaten, vorzugsweise Ethylendiamindisuccinat, sind weitere geeignete Cobuilder. Dabei wird Ethylendiamin-N,N'disuccinat (EDDS) bevorzugt in Form seiner Natrium- oder Magnesiumsalze verwendet. Weiterhin bevorzugt sind in diesem Zusammenhang auch Glycerindisuccinate und Glycerintrisuccinate. Geeignete Einsatzmengen liegen in zeolithhaltigen und/oder silikathaltigen Formulierungen bei 3 bis 15 Gew.-%.

Weitere brauchbare organische Cobuilder sind beispielsweise acetylierte Hydroxycarbonsäuren bzw. deren Salze, welche gegebenenfalls auch in Lactonform vorliegen können und welche mindestens 4 Kohlenstoffatome und mindestens eine Hydroxygruppe sowie maximal zwei Säuregruppen enthalten.

Eine weitere Substanzklasse mit Cobuildereigenschaften stellen die Phosphonate dar. Dabei handelt es sich insbesondere um Hydroxyalkan- bzw. Aminoalkanphosphonate. Unter den Hydroxyalkanphosphonaten ist das 1-Hydroxyethan-1,1-diphosphonat (HEDP) von besonderer Bedeutung als Cobuilder. Es wird vorzugsweise als Natriumsalz eingesetzt, wobei das Dinatriumsalz neutral und das Tetranatriumsalz alkalisch (pH 9) reagiert. Als Aminoalkanphosphonate kommen vorzugsweise Ethylendiamintetramethylenphosphonat (EDTMP), Diethylentriaminpentamethylenphosphonat (DTPMP) sowie deren höhere Homologe in Frage. Sie werden vorzugsweise in Form der neutral reagierenden Natriumsalze, z. B. als Hexanatriumsalz der EDTMP bzw. als Hepta- und Octa-Natriumsalz der DTPMP, eingesetzt. Als Builder wird dabei aus der Klasse der Phosphonate bevorzugt HEDP verwendet. Die Aminoalkanphosphonate besitzen zudem ein ausgeprägtes Schwermetallbindevermögen. Dementsprechend kann es. insbesondere wenn die Mittel auch Bleiche enthalten, bevorzugt sein, Aminoalkanphosphonate, insbesondere DTPMP, einzusetzen, oder Mischungen aus den genannten Phosphonaten zu verwenden.

Darüber hinaus können alle Verbindungen. die in der Lage sind, Komplexe mit Erdalkaliionen auszubilden, als Cobuilder eingesetzt werden.

Als Tenside kommen insbesondere die anionischen und nichtionischen Tenside in Betracht. Während in maschinellen Geschirrspülmitteln üblicherweise lediglich schwachschäumende nichtionische Tenside eingesetzt werden. enthalten Textilwaschmittel in der Regel auch anionische Tenside sowie in geringen Mengen auch ampholytische und zwitterionische Tenside.

Als nichtionische Tenside werden vorzugsweise alkoxylierte, vorteilhafterweise ethoxylierte, insbesondere primäre Alkohole mit vorzugsweise 8 bis 18 C-Atomen und durchschnittlich 1 bis 12 Mol Ethylenoxid (EO) pro Mol Alkohol eingesetzt, in denen der Alkoholrest linear oder bevorzugt in 2-Stellung methylverzweigt sein kann bzw. lineare und methylverzweigte Reste im Gemisch enthalten kann. so wie sie üblicherweise in Oxoalkoholresten vorliegen. Insbesondere sind jedoch Alkoholethoxylate mit linearen Resten aus Alkoholen nativen Ursprungs mit 12 bis 18 C-Atomen, z.B. aus Kokos-, Palm-, Talgfettoder Oleylalkohol, und durchschnittlich 2 bis 8 EO pro Mol Alkohol bevorzugt. Zu den bevorzugten ethoxylierten Alkoholen gehören beispielsweise C₁₂₋₁₄-Alkohole mit 3 EO bis 7 EO, C₉₋₁₁-Alkohol mit 7 EO, C₁₃₋₁₅-Alkohole mit 3 EO, 5 EO. 7 EO oder 8 EO, C₁₂₋₁₈-Alkohole mit 3 EO, 5 EO oder 7 EO und Mischungen aus diesen, wie Mischungen aus C₁₂₋₁₄-Alkohol mit 3 EO und C₁₂₋₁₈-Alkohol mit 7 EO. Die angegebenen Ethoxylierungsgrade stellen statistische Mittelwerte dar. die für ein spezielles Produkt eine ganze oder eine gebrochene Zahl sein können. Bevorzugte Alkoholethoxylate weisen eine eingeengte Homologenverteilung auf (narrow range ethoxylates. NRE). Zusätzlich zu diesen nichtionischen Tensiden können auch Fettalkohole mit mehr als 12 EO eingesetzt werden. Beispiele hierfür sind Talgfettalkohol mit 14 EO, 25 EO, 30 EO oder 40 EO. Auch nichtionische Tenside, die EO- und PO-Gruppen zusammen im Molekül enthalten, sind erfindungsgemäß einsetzbar. Hierbei können Blockcopolymere mit EO-PO-Blockeinheiten bzw. PO-EO-Blockeinheiten eingesetzt werden, aber auch EO-PO-EO-Copolymere bzw. PO-EO-PO-Copolymere. Selbstverständlich sind auch gemischt alkoxylierte Niotenside einsetzbar, in denen EO- und PO-Einheiten nicht blockweise sondern statistisch verteilt sind. Solche Produkte sind durch gleichzeitige Einwirkung von Ethylenund Propylenoxid auf Fettalkohole erhältlich.

Außerdem können als weitere nichtionische Tenside auch Alkylglykoside der allgemeinen Formel RO(G), eingesetzt werden, in der R einen primären geradkettigen oder methylverzweigten, insbesondere in 2-Stellung methylverzweigten aliphatischen Rest mit 8 bis 22, vorzugsweise 12 bis 18 C-Atomen bedeutet und G das Symbol ist, das für eine Glykoseeinheit mit 5 oder 6 C-Atomen, vorzugsweise für Glucose, steht. Der Oligomerisierungsgrad x, der die Verteilung von Monoglykosiden und Oligoglykosiden angibt, ist eine beliebige Zahl zwischen 1 und 10: vorzugsweise liegt x bei 1,2 bis 1.4.

Eine weitere Klasse nichtionischer Tenside sind alkoxylierte. vorzugsweise ethoxylierte oder ethoxylierte und propoxylierte Fettsäurealkylester, vorzugsweise mit 1 bis 4 Kohlenstoffatomen in der Alkylkette, insbesondere Fettsäuremethylester. wie sie beispielsweise in der japanischen Patentanmeldung JP 58/217598 beschrieben sind oder die vorzugsweise nach dem in der internationalen Patentanmeldung WO-A-90/13533 beschriebenen Verfahren hergestellt werden.

Auch nichtionische Tenside vom Typ der Aminoxide, beispielsweise N-Kokosalkyl-N,N-dimethylaminoxid und N-Talgalkyl-N,N-dihydroxyethylaminoxid, der Fettsäurealkanolamide und der Fettaminalkoxylate können geeignet sein. Die Menge dieser nichtionischen Tenside beträgt vorzugsweise nicht mehr als die der ethoxylierten Fettalkohole, insbesondere nicht mehr als die Hälfte davon.

Weitere geeignete Tenside sind Polyhydroxyfettsäureamide der Formel I. 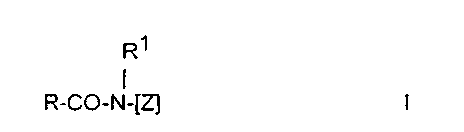 in der RCO für einen aliphatischen Acylrest mit 6 bis 22 Kohlenstoffatomen, R¹ für Wasserstoff, einen Alkyl- oder Hydroxyalkylrest mit 1 bis 4 Kohlenstoffatomen und [Z] für einen linearen'oder verzweigten Polyhydroxyalkylrest mit 3 bis 10 Kohlenstoffatomen und 3 bis 10 Hydroxylgruppen steht. Bei den Polyhydroxyfettsäureamiden handelt es sich um bekannte Stoffe. die üblicherweise durch reduktive Aminierung eines reduzierenden Zukkers mit Ammoniak, einem Alkylamin oder einem Alkanolamin und nachfolgende Acylierung mit einer Fettsäure. einem Fettsäurealkylester oder einem Fettsäurechlorid erhalten werden können.

Zur Gruppe der Polyhydroxyfettsäureamide gehören auch Verbindungen der Formel II, 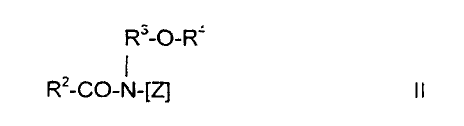 in der R² für einen linearen oder verzweigten Alkyl- oder Alkenylrest mit 7 bis 12 Kohlenstoffatomen, R³ für einen linearen. verzweigten oder cyclischen Alkylrest oder einen Arylrest mit 2 bis 8 Kohlenstoffatomen und R⁴ für einen linearen, verzweigten oder cyclischen Alkylrest oder einen Arylrest oder einen Oxy-Alkylrest mit 1 bis 8 Kohlenstoffatomen steht, wobei C₁₋₄-Alkyl- oder Phenylreste bevorzugt sind und [Z] für einen iinearen Polyhydroxyalkylrest steht, dessen Alkylkette mit mindestens zwei Hydroxylgruppen substituiert ist, oder alkoxylierte. vorzugsweise ethoxylierte oder propxylierte Derivate dieses Restes.

[Z] wird vorzugsweise durch reduktive Aminierung eines Zuckers erhalten, beispielsweise Glucose, Fructose, Maltose, Lactose, Galactose, Mannose oder Xylose.

Als anionische Tenside werden beispielsweise solche vom Typ der Sulfonate und Sulfate eingesetzt. Als Tenside vom Sulfonat-Typ kommen dabei vorzugsweise C₉₋₁₃-Alkylbenzolsulfonate, Olefinsulfonate, d.h. Gemische aus Alken- und Hydroxyalkansulfonaten sowie Disulfonaten, wie man sie beispielsweise aus C₁₂₋₁₈-Monoolefinen mit endoder innenständiger Doppelbindung durch Sulfonieren mit gasförmigem Schwefeltrioxid und anschließende alkalische oder saure Hydrolyse der Sulfonierungsprodukte erhält, in Betracht. Geeignet sind auch Alkansulfonate. die aus C₁₂₋₁₈-Alkanen beispielsweise durch Sulfochlorierung oder Sulfoxidation mit anschließender Hydrolyse bzw. Neutralisation gewonnen werden. Ebenso sind auch die Ester von α-Sulfofettsäuren (Estersulfonate), z.B. die α-sulfonierten Methylester der hydrierten Kokos-, Palmkern- oder Talgfettsäuren geeignet.

Als Alk(en)ylsulfate werden die Alkali- und insbesondere die Natriumsalze der Schwefelsäurehalbester der C₁₂-C₁₈-Fettalkohole, beispielsweise aus Kokosfettalkohol, Taigfettalkohol, Lauryl-, Myristyl-, Cetyl- oder Stearylalkohol oder der C₁₀-C₂₀-Oxoalkohole und diejenigen Halbester sekundärer Alkohole dieser Kettenlängen bevorzugt. Weiterhin bevorzugt sind Alk(en)ylsulfate der genannten Kettenlänge, welche einen synthetischen, auf petrochemischer Basis hergestellten geradkettigen Alkylrest enthalten. Aus waschtechnischem Interesse sind die C₁₂-C₁₆-Alkylsulfate und C₁₂-C₁₅-Alkylsulfate sowie C₁₄-C₁₅-Alkylsulfate bevorzugt. Auch 2.3-Alkylsulfate. welche beispielsweise gemäß den US-Patentschriften 3,234,258 oder 5,075,041 hergestellt werden und als Handelsprodukte der Shell Oil Company unter dem Namen DAN® erhalten werden können, sind geeignete Aniontenside.

Weitere geeignete Aniontenside sind sulfierte Fettsäureglycerinester. Unter Fettsäureglycerinestern sind die Mono-, Di- und Triester sowie deren Gemische zu verstehen, wie sie bei der Herstellung durch Veresterung von einem Monoglycerin mit 1 bis 3 Mol Fettsäure oder bei der Umesterung von Triglyceriden mit 0.3 bis 2 Mol Glycerin erhalten werden.

Bevorzugte sulfierte Fettsäureglycerinester sind dabei die Sulfierprodukte von gesättigten Fettsäuren mit 6 bis 22 Kohlenstoffatomen. beispielsweise der Capronsäure, Caprylsäure, Caprinsäure, Myristinsäure. Laurinsäure. Palmitinsäure. Stearinsäure oder Behensäure.

Auch die Schwefelsäuremonoester der mit 1 bis 6 Mol Ethylenoxid ethoxylierten geradkettigen oder verzweigten C₇₋₂₁-Alkohole, wie 2-Methyl-verzweigte C₉₋₁₁-Alkohole mit im Durchschnitt 3,5 Mol Ethylenoxid (EO) oder C₁₂₋₁₈-Fettalkohole mit 1 bis 4 EO. sind geeignet. Sie werden in Tensidzusammensetzungen bzw. Reinigungsmitteln aufgrund ihres hohen Schaumverhaltens nur in relativ geringen Mengen. beispielsweise in Mengen von 1 bis 5 Gew.-%, eingesetzt.

Weitere geeignete Aniontenside sind auch die Salze der Alkylsulfobernsteinsäure, die aucn als Sulfosuccinate oder als Sulfobernsteinsäureester bezeichnet werden und die Monoester und/oder Diester der Sulfobernsteinsäure mit Alkoholen, vorzugsweise Fettalkoholen und insbesondere ethoxylierten Fettalkoholen darstellen. Bevorzugte Sulfosuccinate enthalten C₈₋₁₈-Fettalkoholreste oder Mischungen aus diesen. Insbesondere bevorzugte Sulfosuccinate enthalten einen Fettalkoholrest, der sich von ethoxylierten Fettalkoholen ableitet, die für sich betrachtet nichtionische Tenside darstellen (Beschreibung siehe unten). Dabei sind wiederum Sulfosuccinate, deren Fettalkohol-Reste sich von ethoxylierten Fettalkoholen mit eingeengter Homologenverteilung ableiten, besonders bevorzugt. Ebenso ist es auch möglich, Alk(en)ylbernsteinsäure mit vorzugsweise 8 bis 18 Kohlenstoffatomen in der Alk(en)ylkette oder deren Salze einzusetzen.

Als weitere anionische Tenside kommen insbesondere Seifen in Betracht, die insbesondere bei höheren pH-Werten eingesetzt werden. Geeignet sind gesättigte und ungesättigte Fettsäureseifen, wie die Salze der Laurinsäure, Myristinsäure, Palmitinsäure, Stearinsaure, hydrierte Erucasäure und Behensäure sowie insbesondere aus natürlichen Fettsäuren, z.B. Kokos-, Palmkern-. Olivenöl- oder Talgfettsäuren. abgeleitete Seifengemische.

Die anionischen Tenside einschließlich der Seifen können in Abhängigkeit vom pH-Wert in Form der freien Säuren oder ihrer Natrium-, Kalium- oder Ammoniumsalze sowie als lösliche Salze organischer Basen. wie Mono-, Di- oder Triethanolamin. vorliegen.

Als zwitterionische Tenside werden solche oberflächenaktiven Verbindungen bezeichnet, die im Molekül mindestens eine quartäre Ammoniumgruppe und mindestens eine -COO⁽⁻⁾oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyloder Acylgruppe sowie das Kokosacylaminoethylhydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer C₈₋₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Geeignete Beispiele für Aminogruppe-haltige Tenside sind die Fettaminalkoxylate.

Bevorzugte Bleichmittel sind H₂O₂ und in Wasser H₂O₂ liefernden Verbindungen, wie Natriumperborattetrahydrat, Natriumperboratmonohydrat, Natriumpercarbonat oder entsprechende Percarbonatsalze, Persilikat, Peroxypyrophosphate. Persulfate, wie Monopersulfat, Harnstoff-Peroxyhydrat. Citratperhydrate, organische Peroxide sowie H₂O₂ liefernde persaure Salze oder Persäuren. wie Perbenzoate, Peroxophthalate. Diperoxyazelainsäure. Phthaloiminopersäuren oder Diperoxydodecandisäure. H₂O₂ wird besonders bevorzugt eingesetzt.

Weitere typische organische Bleichmittel sind die Peroxysäuren. wobei als Beispiele besonders die Alkylperoxysäuren und die Arylperoxysäuren genannt werden können. Geeingete Vertreter sind Dibenzoylperoxid. Peroxybenzoesäure und ihre ringsubstituierten Derivate, wie Alkylperoxybenzoesäuren, aber auch Peroxy-α-Naphtoesäure und Magnesiummonoperphthalat, (b) die aliphatischen oder substituiert aliphatischen Peroxysäuren, wie Peroxylaurinsäure. Peroxystearinsäure, ε-Phthalimidoperoxycapronsäure [Phthaloiminoperoxyhexansäure (PAP)]. o-Carboxybenzamidoperoxycapronsäure, N-nonenylamidoperadipinsäure und N-nonenylamidopersuccinate, und (c) aliphatische und araliphatische Peroxydicarbonsäuren. wie 1.12-Diperoxycarbonsäure, 1.9-Diperoxyazelainsäure, Diperocysebacinsäure. Diperoxybrassylsäure, die Diperoxyphthalsäuren. 2-Decyldiperoxybutan-1.4-disäure, N,N-Terephthaloyl-di(6-aminopercapronsäue) können eingesetzt werden.

Als Bleichmittel in den erfindungsgemäßen Reinigungsmitteln für das maschinelle Geschirrspülen können auch Chlor oder Brom freisetzende Substanzen eingesetzt werden. Unter den geeigneten Chlor oder Brom freisetzenden Materialien kommen beispielsweise heterocyclische N-Brom- und N-Chloramide, beispielsweise Trichlorisocyanursäure, Tribromisocyanursäure. Dibromisocyanursäure und/oder Dichlorisocyanursäure (DICA) und/oder deren Salze mit Kationen wie Kalium und Natrium in Betracht. Hydantoinverbindungen, wie 1,3-Dichlor-5,5-dimethylhydanthoin sind ebenfalls geeignet.

Als Bleichaktivatoren können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Geeignet sind Substanzen, die O- und/oder N-Acylgruppen der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate, insbesondere n-Nonanoyl- oder Isononanoyloxybenzolsulfonat (n- bzw. iso-NOBS). Carbonsäureanhydride. insbesondere Phthalsäureanhydrid, acylierte mehrwertige Alkohole, insbesondere Triacetin, Ethylenglykoldiacetat. 2,5-Diacetoxy-2,5-dihydrofuran, n-Methyl-Morpholinium-Acetonitril-Methylsulfat (MMA), und deren Enolester sowie acetyliertes Sorbitol und Mannitol beziehungsweise deren Mischungen (SORMAN), acylierte Zuckerderivate, insbesondere Pentaacetylglukose (PAG), Pentaacetylfructose, Tetraacetylxylose und Octaacetyllactose sowie acetyliertes, gegebenenfalls N-alkyliertes Glucamin und Gluconolacton, und/oder N-acylierte Lactame. beispielsweise N-Benzoylcaprolactam. Hydrophil substituierte Acylacetale und Acyllactame werden ebenfalls bevorzugt eingesetzt. Auch Kombinationen konventioneller Bleichaktivatoren können eingesetzt werden.

Zusätzlich zu den konventionellen Bleichaktivatoren oder an deren Stelle können auch sogenannte Bleichkatalysatoren enthalten sein. Bei diesen Stoffen handelt es sich um bleichverstärkende Übergangsmetallsalze bzw. Übergangsmetallkomplexe wie beispielsweise Mn-, Fe-, Co-, Ru - oder Mo-Salenkomplexe oder -carbonylkomplexe. Auch Mn-, Fe-, Co-, Ru-, Mo-, Ti-, V- und Cu-Komplexe mit N-haltigen Tripod-Liganden sowie Co-, Fe-, Cu- und Ru-Amminkomplexe sind als Bleichkatalysatoren verwendbar.

Als Enzyme kommen in den erfindungsgemäßen Reinigungsmitteln insbesondere solche aus der Klassen der Hydrolasen wie der Proteasen, Esterasen, Lipasen bzw. lipolytisch wirkende Enzyme. Amylasen. Glykosylhydrolasen und Gemische der genannten Enzyme in Frage. Alle diese Hydrolasen tragen zur Entfernung von Anschmutzungen wie protein-, fett- oder stärkehaltigen Verfleckungen bei. Zur Bleiche können auch Oxidoreduktasen eingesetzt werden. Besonders gut geeignet sind aus Bakterienstämmen oder Pilzen wie Bacillus subtilis, Bacillus licheniformis, Streptomyceus griseus, Coprinus Cinereus und Humicola insolens sowie aus deren gentechnisch modifizierten Varianten gewonnene enzymatische Wirkstoffe. Vorzugsweise werden Proteasen vom Subtilisin-Typ und insbesondere Proteasen, die aus Bacillus lentus gewonnen werden, eingesetzt. Dabei sind Enzymmischungen, beispielsweise aus Protease und Amylase oder Protease und Lipase bzw. lipolytisch wirkenden Enzymen oder aus Protease, Amylase und Lipase bzw. lipolytisch wirkenden Enzymen oder Protease, Lipase bzw. lipolytisch wirkenden Enzymen, insbesondere jedoch Protease und/oder Lipase-haltige Mischungen bzw. Mischungen mit lipolytisch wirkenden Enzymen von besonderem Interesse. Beispiele für derartige lipolytisch wirkende Enzyme sind die bekannten Cutinasen. Auch Peroxidasen oder Oxidasen haben sich in einigen Fällen als geeignet erwiesen. Zu den geeigneten Amylasen zählen insbesondere alpha-Amylasen. Iso-Amylasen, Pullulanasen und Pektinasen.

Die Enzyme können an Trägerstoffe adsorbiert oder in Hüllsubstanzen eingebettet sein, um sie gegen vorzeitige Zersetzung zu schützen. Der Anteil der Enzyme, Enzymmischungen oder Enzymgranulate kann beispielsweise etwa 0,1 bis 5 Gew.-%, vorzugsweise 0,5 bis etwa 4,5 Gew.-% betragen.

Farb- und Duftstoffe können den erfindungsgemäßen maschinellen Geschirrspülmitteln zugesetzt werden, um den ästhetischen Eindruck der entstehenden Produkte zu verbessern und dem Verbraucher neben der Leistung ein visuell und sensorisch "typisches und unverwechselbares" Produkt zur Verfügung zu stellen. Als Parfümöle bzw. Duftstoffe können einzelne Riechstoffverbindungen, z.B. die synthetischen Produkte vom Typ der Ester, Ether, Aldehyde, Ketone. Alkohole und Kohlenwasserstoffe verwendet werden. Riechstoffverbindungen vom Typ der Ester sind z.B. Benzylacetat. Phenoxyethylisobutyrat, p-tert.-Butylcyclohexylacetat. Linalylacetat. Dimethylbenzylcarbinylacetat, Phenylethylacetat. Linalylbenzoat. Benzylformiat, Ethylmethylphenylglycinat. Allylcyclohexylpropionat. Styrallylpropionat und Benzylsalicylat. Zu den Ethern zählen beispielsweise Benzylethylether, zu den Aldehyden z.B. die linearen Alkanale mit 8-18 C-Atomen. Citral, Citronellal. Citronellyloxyacetaldehyd, Cyclamenaldehyd, Hydroxycitronellal, Lilial und Bourgeonal, zu den Ketonen z.B. die Jonone. α-Isomethylionon und Methylcedrylketon, zu den Alkoholen Anethol, Citronellol, Eugenol, Geraniol, Linalool, Phenylethylalkohol und Terpineol, zu den Kohlenwasserstoffen gehören hauptsächlich die Terpene wie Limonen und Pinen. Bevorzugt werden jedoch Mischungen verschiedener Riechstoffe verwendet, die gemeinsam eine ansprechende Duftnote erzeugen. Solche Parfümöle können auch natürliche Riechstoffgemische enthalten, wie sie aus pflanzlichen Quellen zugänglich sind. z.B. Pine-, Citrus-, Jasmin-. Patchouly-, Rosen- oder Ylang-Ylang-Öl. Ebenfalls geeignet sind Muskateller, Salbeiöl, Kamillenöl, Nelkenöl, Melissenöl, Minzöl, Zimtblätteröl, Lindenblütenöl, Wacholderbeeröl, Vetiveröl, Olibanumöl, Galbanumöl und Labdanumöl sowie Orangenblütenöl, Neroliol. Orangenschalenöl und Sandelholzöl.

Um den ästhetischen Eindruck der erfindungsgemäß hergestellten Formkörper zu verbessern. können sie (oder Teile davon) mit geeigneten Farbstoffen eingefärbt werden. Bevorzugte Farbstoffe, deren Auswahl dem Fachmann keinerlei Schwierigkeit bereitet, besitzen eine hohe Lagerstabilität und Unempfindlichkeit gegenüber den übrigen Inhaltsstoffen der Mittel und gegen Licht sowie keine ausgeprägte Substantivität gegenüber den mit den Mitteln zu behandelnden Substraten wie Glas. Keramik oder Kunststoffgeschirr, oder Textilien, um diese nicht anzufärben.

Werden die erfindungsgemäß hergestellten Reinigungsmittel zum maschinellen Geschirrspülen eingesetzt, können sie zum Schutze des Spülgutes oder der Maschine Korrosionsinhibitoren enthalten, wobei besonders Silberschutzmittel eine besondere Bedeutung haben. Allgemein können vor allem Silberschutzmittei ausgewählt aus der Gruppe der Triazole, der Benzotriazole, der Bisbenzotriazole, der Aminotriazole, der Alkylaminotriazole und der Übergangsmetallsalze oder -komplexe eingesetzt werden. Besonders bevorzugt zu verwenden sind Benzotriazol und/oder Alkylaminotriazol. Man findet in Reinigerformulierungen darüber hinaus häufig aktivchlorhaltige Mittel, die das Korrodieren der Silberoberfläche deutlich vermindern können. In chlorfreien Reinigern werden besonders Sauerstoff- und stickstoffhaltige organische redoxaktive Verbindungen. wie zwei- und dreiwertige Phenole, z. B. Hydrochinon. Brenzkatechin, Hydroxyhydrochinon. Gallussäure, Phloroglucin, Pyrogallol bzw. Derivate dieser Verbindungsklassen. Auch salz- und komplexartige anorganische Verbindungen, wie Salze der Metalle Mn, Ti, Zr, Hf, V, Co und Ce finden häufig Verwendung. Bevorzugt sind hierbei die Übergangsmetallsalze, die ausgewählt sind aus der Gruppe der Mangan und/oder Cobaltsalze und/oder -komplexe, besonders bevorzugt der Cobalt(ammin)-Komplexe. der Cobalt(acetat)-Komplexe. der Cobalt-(Carbonyl)-Komplexe, der Chloride des Cobalts oder Mangans und des Mangansulfats. Ebenfalls können Zinkverbindungen zur Verhinderung der Korrosion am Spülgut eingesetzt werden.

In einer weiteren bevorzugten Ausführungsform ist der erfindungsgemäß hergestellte Formkörper ein maschinelles Geschirrspülmittel, das eine Kavität aufweist. In die Kavität können Füllungen (im folgenden auch als Kern bezeichnet) eingebracht werden, die vorzugsweise Aktivstoffe enthalten, welche erst in einem Wasch- oder Spülgang nach der eigentlichen Geschirrreinigung freigesetzt werden. vorzugsweise im Klarspülgang.

Der Kern enthält in dieser Ausführungsform als Aktivstoffe vorzugsweise einen oder mehrere Stoffe aus der Gruppe der Tenside einschließlich Kiarspültenside, Bleichmittel, Bleichaktivatoren, Korrosionsinhibitoren, Belagsinhibitoren. Duftstoffe und/oder Cobuilder, wobei der Einsatz der weiteren oben genannten Aktivstoffe im Kern nicht ausgeschlossen sein soll.

Es können auch Duftstoffe in den Kern eingearbeitet werden. was zu einem Dufteindruck beim Öffnen der Maschine (siehe oben) führt.

In einer weiteren bevorzugten Ausführungsform ist der erfindungsgemäße Formkörper ein Textilwaschmittel, das eine Kavität aufweist. In die Kavität können Füllungen eingebracht werden, die vorzugsweise Aktivstoffe enthalten, welche erst in einem Wasch- oder Spüigang nach der eigentlichen Textilwäsche freigesetzt werden. vorzugsweise in einem Klarspülgang. Die im Kern eingearbeiteten Aktivstoffe weisen vorzugsweise textilpflegende Eigenschaften auf.

Der Kern enthält in dieser Ausführungsform als Aktivstoffe vorzugsweise eine oder mehrere Stoffe aus der Gruppe der anionischen, nichtionischen, kationischen und/oder amphoteren bzw. zwitterionischen Tenside, der kationischen und/oder nichtionischen Textilweichmachern, Schauminhibitoren, Parfümöle, Farbstoffe, Konservierungsmittel, schmutzablösende Polymere, Antistatika und/oder Bügelhilfsmittel, wobei der Einsatz der weiteren oben genannten Aktivstoffe im Kern nicht ausgeschlossen sein soll.

Die erfindungsgemäß hergestellten Formkörper können auch im Bereich der kosmetischen oder pharmazeutischen Produkte eingesetzt werden, wobei die Ausgangssubstanzen die kosmetischen oder pharmazeutischen Wirkstoffe darstellen. Das erfindungsgemäße Verfahren ist ebenfalls zur Herstellung von Formkörpern für Klebstoffe, insbesondere Klebstoffe auf Wasserbasis, wie Tapetenkleister geeignet. Noch weitere Einsatzgebiete sind sämtliche Bereiche, in denen Wirkstoffe dosiert werden müssen, z. B. in der Landwirtschaft und im Gartenbau, wie Düngemittel und Pestizide, Herbizide, Mittel zur Wasseraufbereitung, Brennstofftabletten, Katalysatoren, die in Form von Formkörpern eingesetzt werden.

### Beispiele

### Beispiel 1:

Es wurde ein Waschmittelformkörper auf folgende Weise hergestellt:
Zunächst wurde in einem Mischer ein Granulat folgender Zusammensetzung hergestellt:

| **Inhaltsstoff** | **Anteil / Gew.%** |
|---|---|
| ABS (Na-Salz) | 20 |
| C12-18 Fettalkohol + 7EO | 6 |
| C12-18 Fettalkoholsulfat Na | 4 |
| Zeolith A | 30 |
| Soda | 15 |
| Na-Wasserglas | 6 |
| Polycarboxylat | 5 |
| Wasser | 10 |
| Na-Sulfat | ad 100 |

Nach Trocknen des Granulats in einem Wirbelschichttrockner auf den angegebenen Wassergehalt und Absieben auf Teilchengrößen < 0,8 mm wurde wiederum im Mischer eine Mischung der folgenden Zusammensetzung hergestellt:

| **Inhaltsstoff** | **Anteil / Gew.%** |
|---|---|
| Granulat | 65 |
| Na-Percarbonat, gecoatet | 15 |
| TAED | 6 |
| Enzymgranulat | 3 |
| Ubliche Hilfsstoffe (Schauminhibitor-Granulat. Parfümöl etc.) | 5 |
| Na-Sulfat | ad 100 |

Diese Mischung wurde mit 70%-iger KOH im Mischer gecoatet. so dass 2% bzw. 4% reines KOH bezogen auf die Mischung auf den Oberflächen der Granula vorlagen. Das überschüssige Wasser wird vom Granulat aufgenommen. Von diesem gecoateten Granulat wurden 25g in eine Matrize mit 40 mm Durchmesser eingefüllt und leicht mit einem Stempel (100 N) angedrückt. Anschließend wurde im leichten Strom CO₂ durch die Matrize geleitet. Die Reaktion kann durch Messung der Temperatur verfolgt werden. der Formling erwärmt sich während der Reaktion auf ca. 40°C. Das Überschreiten des Temperaturmaximums an der Unterseite des Formkörpers zeigt das Ende der Reaktion an. Dies ist bei der verwendeten Versuchsanordnung nach ca. 2-5 min der Fall. Der Formling kann dann mit einem Stempei aus der Matrize herausgedrückt werden.

### Beispiel 2:

es wurde ein Formkörper für ein maschinelies Geschirrspülmittel auf folgende Weise hergestellt:
Zunächst wird ein Gemisch der folgenden Zusammensetzung hergestellt:

| **Inhaltsstoff** | **Anteil / Gew.%** |
|---|---|
| Na-Tripolyphosphat | 50 |
| Soda | 15 |
| Na-Silikat | 5 |
| Polycarboxylat | 1 |
| Phosphonat | 1 |
| nichtionische Tenside | 2 |
| Na-Perborat Monohydrat | 10 |
| TAED | 2 |
| Enzymgranulat | 4 |
| Übliche Hilfsstoffe, Wasser, Salze | ad 100 |

Diese Mischung wurde nach Absiebung auf < 0.8 mm mit 50% iger NaOH im Mischer gecoatet, so dass 5% bzw. 7% reines NaOH bezogen auf die Mischung auf den Oberflächen der Granula vorlagen. Von diesem beschichteten Granulat wurden 25g in eine Matrize eingefüllt und leicht mit einem Stempel (100 N) angedrückt. Anschließend wurde wie in Beispiel 1 weiter verfahren.

### Beispiel 3:

Es wurde ein Formkörper für ein maschinelles Geschirrspülmittel mit integriertem Klarspüier auf folgende Weise hergestellt:
Zunachst wird der Vorformling analog Beispiel 2 hergestellt mit dem Unterschied, dass der Stempel oder der Boden der Matrize. die der Formgebung dienen eine Ausbuchtung besitzen. Anschließend werden die Formkörper wie in den vorigen Beispielen durch die Einleitung von CO₂ verfestigt. Nach dem Herausnehmen aus der Matrize erhält man einen Formkörper, der an einer Fläche eine Kavität aufweist. In diese Kavität wird 1 g einer Schmelze folgender Zusammensetzung dosiert:

| **Inhaltsstoff** | **Anteil / Gew.%** |
|---|---|
| Polytergent SLF 18 B 45 (Olin Chemicals) | 45 |
| Paraffin, Smp. 60 - 63°C | 50 |
| Emulgator | 5 |

Alternativ kann auch ein gegossener Kern beliebiger Form separat hergestellt werden und in der Kavität montiert werden.

## Patentansprüche

1. Verfahren zur Herstellung von Formkörpern enthaltend einen oder mehrere Aktivstoffe, worin pulverförmige bis granulare Ausgangskomponenten in Form gebracht und anschließend verfestigt werden indem eine Komponente A und eine Komponente B miteinander zur Reaktion gebracht werden, wobei die Komponenten A und B mit den Ausgangskomponenten vermischt, darauf aufgebracht oder nach dem Informbringen zugesetzt werden, wobei das Verfestigen durch das gebildete Reaktionsprodukt aus den Komponenten A und B erfolgt, **dadurch gekennzeichnet, dass** die Ausgangskomponenten nachdem sie in Form gebracht worden sind mit einem Druck bis zu 1 kN angedrückt werden oder durch Vibration verdichtet werden.

2. Verfahren nach Anspruch 1, **dadurch gekennzeichnet, dass** die Ausgangskomponenten mit der Komponente A vermischt oder beschichtet sind.

3. Verfahren nach Anspruch 1 oder 2, **dadurch gekennzeichnet, dass** die Komponente A ausgewählt ist aus Alkalihydroxiden, Erdalkalihydroxiden, Alkalisilikaten organischen oder anorganischen Säuren oder sauren Salzen, Hydrate bildenden wasserfreien Salzen oder Hydratwasser-haltigen Salzen.

4. Verfahren nach einem der Ansprüche 1 bis 3, **dadurch gekennzeichnet, dass** die Komponente B ausgewählt ist aus CO₂, NH₃, Wasserdampf, Hydratwasser-haltigen Salzen, Hydrate bildenden wasserfreien Salzen, SO₂, SO₃, HCl, HBr.

5. Verfahren nach einem der Ansprüche 3 und 4, **dadurch gekennzeichnet, dass** die Komponente A ausgewählt ist aus NaOH, KOH oder Ca(OH)₂ und die Komponente B CO₂ ist.

6. Verfahren nach einem der Ansprüche 1 bis 5, **dadurch gekennzeichnet, dass** die Komponente B ein Gas ist und ein Vorformling von dem Gas durchströmt wird oder dieser unter Überdruck dem Gas ausgesetzt wird, oder in eine Atmosphäre diese Gases eingebracht wird oder dass man zunächst das Gas durch den Vorformling strömen lässt, um Luft zu verdrängen und dann in eine Atmosphäre aus dem Gas einbringt.

7. Verfahren nach einem der Ansprüche 1 bis 6, **dadurch gekennzeichnet, dass** mehrphasige und/oder mehrphasige Formkörper hergestellt werden.

8. Verfahren nach Anspruch 7, **dadurch gekennzeichnet, dass** die einzelnen Phasen unterschiedliche Härten und/oder Zerfallszeiten aufweisen.

9. Verfahren nach einem der Ansprüche 1 bis 8, **dadurch gekennzeichnet, dass** die Formkörper eine Kavität aufweisen, in die geeignete Füllungen, eingebracht werden können.

10. Verfahren nach Anspruch 9, **dadurch gekennzeichnet, dass** die Füllung(en) in den Vorformling eingearbeitet wird und anschließend die Verfestigung erfolgt.

11. Verfahren nach einem der Ansprüche 1 bis 10, **dadurch gekennzeichnet, dass** der Formkörper ein maschinelles Geschirrspülmittel oder Textilwaschmittel ist.

12. Verfahren nach Anspruch 11, **dadurch gekennzeichnet, dass** als Aktivstoffe Buildermaterialien, Tenside, Bleichmittel, Bleichaktivatoren, Enzyme, Enzyrnstabilisatoren, Korrosionsinhibitoren, Belag sinhibitoren, Komplexbildner, anorganische Salze, Vergrauungsinhibitoren Schauminhibitoren, Silikonöle, Soil-release-Verbindungen, Farbübertragungsinhibioren, Salze von Polyphosphonsäuren, optische Aufheller, Fluoreszenzmittel, Desinfektionsmittel, Duftstoffe, Fartstoffe, Antistatika, Bügelhilfsmitteln, Phobier- und Imprägniermittel, Quell- und Schiebefestmittel, UV-Absorbern oder deren Gemische enthalten sind.

13. Verfahren nach einem der Ansprüche 11 oder 12, **dadurch gekennzeichnet, dass** der Formkörper ein maschinelles Geschirrspülmittel ist und eine Kavität aufweist.

14. Verfahren nach Anspruch 13, **dadurch gekennzeichnet, dass** in die Kavität Füllungen eingebracht werden, die Aktivstoffe enthalten, welche erst in einem Wasch- oder Spülgang nach der eigentlichen Geschirrreinigung freigesetzt werden, vorzugsweise im Klarspülgang.

15. Verfahren nach Anspruch 14, **dadurch gekennzeichnet, dass** die Aktivstoffe ausgewählt sind aus Tensiden einschließlich Klarspültensiden, Bleichmitteln, Bleichaktivatoren, Korrosionsinhibitoren, Belagsinhibitoren, Duftstoffen und/oder Cobuildern.

16. Verfahren nach einem der Ansprüche 11 oder 12, **dadurch gekennzeichnet, dass** der Formkörper ein Textilwaschmittel ist und eine Kavität aufweist

17. Verfahren nach Anspruch 16, **dadurch gekennzeichnet, dass** in die Kavität Füllungen eingebracht werden, die Aktivstoffe enthalten, welche erst in einem Wasch- oder Spülgang nach der eigentlichen Textilwäsche freigesetzt werden, vorzugsweise in einem Klarspülgang.

18. Verfahren nach Anspruch 14, **dadurch gekennzeichnet, dass** die Aktivstoffe ausgewählt sind aus anionischen, nichtionischen, kationischen und/oder amphoteren bzw. zwitterionischen Tensiden, kationischen und/oder nichtionischen Textilweichmachem, Schauminhibitoren, Parfümöle, Farbstoffe, Konservierungsmittel, schmutzablösende Polymere, Antistatika und/oder Bügelhilfsmitteln.

19. Verfahren nach einem der Ansprüche 1 bis 10, **dadurch gekennzeichnet, dass** der Formkörper ein Desinfektionsmittel enthält.

20. Verfahren nach einem der Ansprüche 1 bis 10, **dadurch gekennzeichnet, dass** der Formkörper kosmetische und/oder pharmazeutische Wirkstoffe enthält.

21. Verfahren nach einem der Ansprüche 1 bis 10, **dadurch gekennzeichnet, dass** der Formkörper als Aktivstoff Klebstoffe, insbesondere Klebstoffe auf Wasserbasis, enthält.

22. Verfahren nach Anspruch 21, **dadurch gekennzeichnet, dass** der Formkörper als Klebstoff Tapetenkleister enthält.

23. Verfahren nach einem der Ansprüche 1 bis 10, **dadurch gekennzeichnet, dass** als Aktivstoffe chemische und biologische Düngemittel, Pestizide, Herbizide, Komponenten zur Wasseraufbereitung, Katalysatoren, und Brennstoffe enthalten sind.

## Claims

1. A process for producing tablets comprising one or more active substances, in which pulverulent to granular starting components are shaped and then solidified, subsequently solidified by reacting a component A and a component B with one another, components A and B being mixed with the starting components, applied to them or added after shaping, solidification taking place by the reaction product formed from components A and B, **characterized in that,** after they have been shaped, the starting components are pressed down with a pressure of up to 1 kN or are compacted by vibration.

2. The process of claim 1, **characterized in that** the starting components are mixed or coated with component A.

3. The process of claim 1 or 2, **characterized in that** component A is selected from alkali metal hydroxides, alkaline earth metal hydroxides, alkali metal silicates organic or inorganic acids or acidic salts, anhydrous salts which form hydrates or salts containing water of hydration.

4. The process of one of claims 1 to 3, **characterized in that** component B is selected from CO₂, NH₃, water vapor, salts containing water of hydration, anhydrous salts which form hydrates, SO₂, SO₃, HCl, HBr.

5. The process of one of claims 3 and 4, **characterized in that** component A is selected from NaOH, KOH or Ca(OH)₂ and component B is CO₂.

6. The process of one of claims 1 to 5, **characterized in that** component B is a gas and a gas flows through a preform or said preform is exposed to the gas under superatmospheric pressure, or is introduced into an atmosphere of this gas, or **in that** first of all the gas is caused to flow through the preform in order to expel air and the preform is then introduced into an atmosphere comprising the gas.

7. The process of one of claims 1 to 6, **characterized in that** multiphase and/or multiphase tablets are produced.

8. The process of claim 7, **characterized in that** the individual phases have different hardnesses and/or disintegration times.

9. The process of one of claims 1 to 8, **characterized in that** the tablets have a cavity into which suitable fillings can be introduced.

10. The process of claim 9, **characterized in that** the filling(s) is incorporated into the preform and then solidification takes place.

11. The process of one of claims 1 to 10, **characterized in that** the tablet is a machine dishwashing detergent or laundry detergent.

12. The process of claim 11, **characterized in that** active substances present comprise builder materials, surfactants, bleach activators, enzymes, enzyme stabilizers, corrosion inhibitors, scale inhibitors, complexing agents, inorganic salts, graying inhibitors, foam inhibitors, silicone oils, soil release compounds, color transfer inhibitors, salts of polyphosphonic acids, optical brighteners, fluorescence agents, disinfectants, fragrances, dyes, antistats, easy-iron agents, repellants and impregnants, swelling and nonslip agents, UV absorbers or mixtures thereof.

13. The process of one of claims 11 or 12, **characterized in that** the tablet is a machine dishwashing tablet and has a cavity.

14. The process of claim 13, **characterized in that** fillings are introduced into the cavity which comprise active substances which are released only in a wash cycle or rinse cycle after the actual cleaning of the ware, preferably in the clear-rinse cycle.

15. The process of claim 14, **characterized in that** the active substances are selected from surfactants, including rinse-aid surfactants, bleaches, bleach activators, corrosion inhibitors, scale inhibitors, fragrances and/or cobuilders.

16. The process of one of claims 11 or 12, **characterized in that** the tablet is a laundry detergent and has a cavity.

17. The process of claim 16, **characterized in that** fillings are introduced into the cavity which comprise active substances which are not released until a wash cycle or rinse cycle after the actual washing of the textile, preferably in a clear-rinse cycle.

18. The process of claim 14, **characterized in that** the active substances are selected from anionic, nonionic, cationic and/or amphoteric or zwitterionic surfactants, cationic and/or nonionic textile softeners, foam inhibitors, perfume oils, dyes, preservatives, soil release polymers, antistats and/or easy-iron agents.

19. The process of one of claims 1 to 10, **characterized in that** the tablet comprises a disinfectant.

20. The process of one of claims 1 to 10, **characterized in that** the tablet comprises active cosmetic and/or pharmaceutical substances.

21. The process of one of claims 1 to 10, **characterized in that** the tablet comprises as active substance adhesives, especially water-based adhesives.

22. The process of claim 21, **characterized in that** the tablet comprises wallpaper paste as adhesive.

23. The process of one of claims 1 to 10, **characterized in that** active substances present comprise chemical and biological fertilizers, pesticides, herbicides, components for water preparation, catalysts and fuels.

## Revendications

1. Procédé pour la préparation de corps moulés contenant une ou plusieurs substances actives, dans lequel on met en forme desc omposants de départ pulvérulents à granuleux avant de les solidifier en faisant réagir un composant A et un composant B l'un avec l'autre, les composants A et B étant mélangés avec les composants de départ, appliqués sur ces derniers ou y étant ajoutés après la mise en forme, la solidification ayant lieu via lep roduit réactionnel formé à partir des composants A et B, **caractérisé en ce que** lesc omposants de dé art, aprèsl eur mise en forme, sont soumis à une compression avec une pression s'élevant jusqu'à 1 kN ou encore à une densification par vibration.

2. Procédé selon la revendication 1, **caractérisé en ce que** les composants de départ sontm élangés ou enduits avec le composantA.

3. Procédé selon la revendication 1 ou 2, **caractérisé en ce que** le composant A est choisi parmi le groupe comprenant des hydroxydes de métaux alcalins, des hydroxydes de métaux alcalino-terreux, des silicates de métaux alcalins, des acides organiques ou inorganiques ou des sels acides, des sels anhydres formant des hydrates ou encore des sels contenantd e l'eau d'hydratation.

4. Procédé selon l'une quelconque des revendications 1 à 3, **caractérisé en ce que** le composant B est choisi parmi le groupe comprenant du CO₂, du NH₃, de la vapeur d'eau, des sels contenant de l'eau d'hydratation, des sels anhydres formant des hydrates, du SO₂, du SO₃, du HCl,d u HBr.

5. Procédé selon l'une quelconque des revendications 3 et 4, **caractérisé en ce que** le composant A est choisi parmi le groupe comprenant du NaOH, du KOH ou du Ca(OH)₂ et le composant B représente du dioxyde de carbone.

6. Procédé selon l'une quelconque des revendications 1 à 5, **caractérisé en ce que** le composant B est un gaz et un préformé est traversé par le gaz ou bien est exposé au gaz dans des conditions de surpression, ou encore est introduit dans une atmosphère de ce gaz ou bien **en ce qu'**on laisse d'abord s'écouler le gaz à travers le préformé pour repousser l'air avant d'introduire leditp réformé dans une atmosphère constituée du gaz.

7. Procédé selon l'une quelconque des revendications 1 à 6, **caractérisé en ce qu**'on prépare des corps moulés monophasiques et/ou polyphasiques.

8. Procédé selon la revendication 7, **caractérisé en ce que** les phases individuelles présentent des duretés différentes et/ou des temps de désintégration différents.

9. Procédé selon l'une quelconque des revendications 1 à 8, **caractérisé en ce que** lesc orps moulés présentent une cavité dans laquelle on peut introduire des matières de charge appropriées.

10. Procédé selon la revendication 9, **caractérisé en ce que** la ou les matières de charges sont incorporées dans le préformé avant de procéder à la solidification.

11. Procédé selon l'une quelconque des revendications 1 à 10, **caractérisé en ce que** le corps moulé est un produit de lavage mécanique de la vaisselle ou un produit de lavage des textiles.

12. Procédé selon lar evendication1 1, **caractérisé en ceq uel** es corps moulés contiennent, à titre de substances actives, des matières faisant office de « builder », des agents tensioactifs, des agents de blanchiment, des activateurs du blanchiment, des enzymes, des stabilisateurs enzymatiques, des inhibiteurs de la corrosion, des inhibiteurs du dépôt, des formateurs de complexes, des sels inorganiques, des inhibiteurs du grisonnement, des inhibiteurs de mousse, des huiles des ilicone, des composés d'élimination de la saleté, des inhibiteurs du transfert de colorants, des sels d'acides polyphosphoniques, des agents de blanchiment optique, des agents fluorescents, des désinfectants, des arômes, des colorants, des agents antistatiques, des adjuvants du repassage, des agents de répulsion et d'imprégnation, des agents de gonflement et de résistance au glissement, des absorbants de l'ultraviolet, ou encore leurs mélanges.

13. Procédé selon l'une quelconque des revendications 11 ou 12, **caractérisé en ce que** le corps moulé est un produit de lavage mécanique de la vaisselle etp résente une cavité.

14. Procédé selon la revendication 13, **caractérisé en ce qu'**on introduit dans la cavité des matières de charges qui contiennent des substances actives qui ne sont libérées, au cours d'un programme de lavage ou d'un programme de rinçage, qu'après le nettoyage proprement dit de la vaisselle,d e préférence au cours du programme de rinçage.

15. Procédé selon la revendication 14, **caractérisé en ce que** les substances actives sont choisies parmi le groupe comprenant des agents tensioactifs y compris des agents tensioactifs pour le rinçage, des agents de blanchiment, des activateurs du blanchiment, des inhibiteurs de la corrosion, des inhibiteurs du dé ôt, des arômes et/ou des « cobuilder ».

16. Procédé selon l'une quelconque des revendications 11 ou 12, **caractérisé en ce que** le corps moulé est un produit de lavage des textiles et présente une cavité.

17. Procédé selon la revendication 16, **caractérisé en ce qu'**on introduit dans la cavité des matières de charges qui contiennent des substances actives qui ne sont libérées, au cours d'un programme de lavage ou d'un programme de rinçage, qu'après le nettoyage des textiles proprement dit, de préférence lors du programme de rinçage.

18. Procédé selon la revendication 17, **caractérisé en ce que** les substances actives sont choisies parmi le groupe comprenant des agents tensioactifs anioniques, non ioniques, cationiques et/ou amphotères, respectivement zwitterioniques, des agents cationiques et/ou non ioniques assouplissant les textiles, des inhibiteurs de mousse, dese ssencesd e parfums, des colorants, des conservants, des polymères repoussant la saleté, des agents antistatiques et/ou des adjuvants du repassage.

19. Procédé selon l'une quelconque des revendications 1 à 10, **caractérisé en ce que** le corps moulé contientu n désinfectant.

20. Procédé selon l'une quelconque des revendications 1 à 10, **caractérisé en ce que** le corps moulé contient des substances actives cosmétiques et/ou pharmaceutiques.

21. Procédé selon l'une quelconque des revendications 1 à 10, **caractérisé** en ce quel ec orps moulé contient, à titre de substance active, des adhésifs,e n particulier des adhésifs à base d'eau.

22. Procédé selon la revendication 21, **caractérisé en ce que** le corps moulé contient, à titre d'adhésif, de la colle à tapisser.

23. Procédé selon l'une quelconque des revendications 1 à 10, **caractérisé en ce que** les corps moulés contiennent, à titre de substances actives, des engrais chimiques et biologiques, des pesticides, des herbicides, des composants pour le traitement de l'eau, des catalyseurs et des combustibles.
